# EUROPÄISCHE PATENTSCHRIFT

(11) **EP 0 569 947 B1**
(45) Veröffentlichungstag und Bekanntmachung des Hinweises auf die Patenterteilung: **01.03.1995**
(21) Anmeldenummer: 93107671.5
(22) Anmeldetag: 11.05.1993
(51) Int. Cl.: C07D 213/61

(54) **Verfahren zur Herstellung von 2-Chlor-5-chlormethyl-pyridin**
Process for the production of 2-chloro-5-chloromethyl-pyridine
Procédé pour la préparation de 2-chloro-5-chloro-méthylpyridine

(30) Priorität: 12.05.1992 CH 1518/92
(43) Veröffentlichungstag der Anmeldung: 18.11.1993
(73) Patentinhaber: LONZA AG, CH-3945 Gampel/Wallis (CH)
(72) Erfinder: Werbitzky, Oleg, Dr., Visp, (Kanton Wallis) (CH); Studer, Philipp, Visp, (Kanton Wallis) (CH)
(74) Vertreter: Weinhold, Peter, Dr.

(56) Entgegenhaltungen:
- EP-A- 0 172 595
- EP-A- 0 260 485
- EP-A- 0 370 317
- EP-A- 0 373 463
- EP-A- 0 373 464
- EP-A- 0 393 453
- EP-A- 0 458 109

## Beschreibung

Die Erfindung betrifft ein neues Verfahren zur Herstellung von 2-Chlor-5-chlormethyl-pyridin der Formel 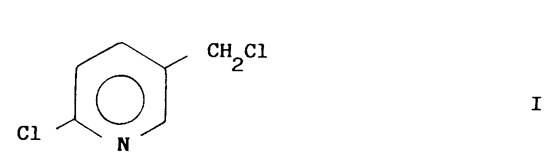
ausgehend von 6-Hydroxynikotinsäure.

2-Chlor-5-chlormethyl-pyridin ist ein wichtiges Zwischenprodukt zur Herstellung von Insektiziden (EP-A 373 464).

Bisher sind mehrere aufwendige Verfahren zur Herstellung von 6-Chlor-3-chlormethyl-pyridin, welches im folgenden als CCPM abgekürzt wird, bekannt.

Beispielsweise beschreibt sowohl die EP-A 373 463 als auch die EP-P 373 464 ein Verfahren zur Herstellung von CCPM, ausgehend von Nikotinsäure.

Beide Anmeldungen beschreiben ein 5-stufiges Verfahren nach folgendem Reaktionsschema:

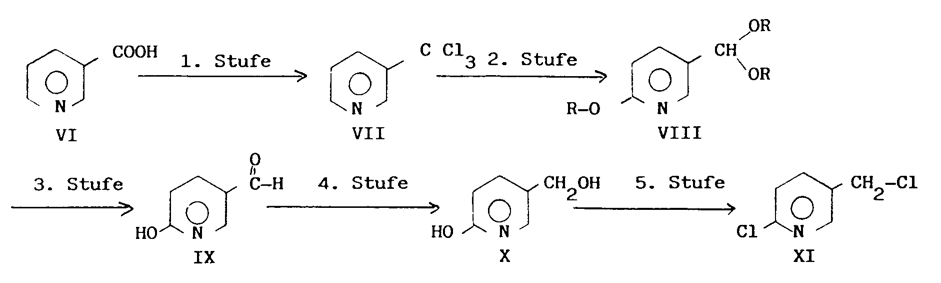

Die Nachteile dieser beiden Verfahren bestehen darin, dass das gewünschte Produkt nur durch eine aufwendige 5-stufige Synthese hergestellt wird und demnach CCPM bezüglich des Eduktes Nikotinsäure in geringer Ausbeute erhalten wird. Ein weiterer Nachteil besteht darin, dass bei diesen Verfahren eine grosse Menge an Salzen als Abfall anfallen.

Aufgabe der vorliegenden Erfindung war, diese Nachteile zu beseitigen und ein wirtschaftlicheres und technisch weniger aufwendiges Verfahren zur Herstellung von CCPM zur Verfügung zu stellen.

Die Aufgabe wurde mit dem erfindungsgemässen Verfahren gemäss Patentanspruch 1 gelöst.

Erfindungsgemäss wird das Verfahren derart durchgeführt, dass man in der ersten Stufe 6-Hydroxynikotinsäure der Formel 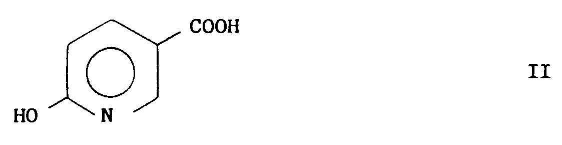
mit einem Säurechlorid zum 6-Hydroxynikotinoylchlorid der Formel 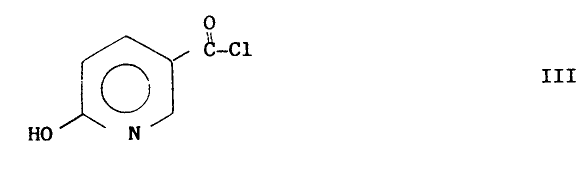
umsetzt, dieses in der zweiten Stufe katalytisch mit Wasserstoff zum 6-Hydroxynikotinsäurealdehyd der Formel 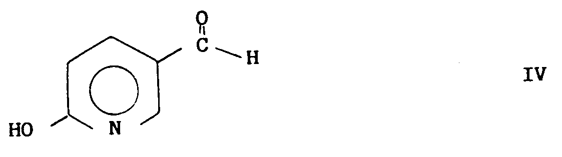
hydriert, dann in der dritten Stufe katalytisch mit Wasserstoff zum 2-Hydroxy-5-hydroxymethylpyridin der Formel 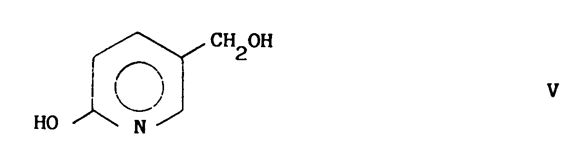
hydriert und dieses dann weiter in der vierten Stufe zum Endprodukt gemäss Formel I chloriert.

Ein besonderer Vorteil des erfindungsgemässen Verfahrens ist, dass das Edukt, die 6-Hydroxynikotinsäure unter einfachen Reaktionsbedingungen mit leicht zugänglichen Reagenzien in CCPM überführt werden kann.

Die erste Stufe, die Umsetzung von 6-Hydroxynikotinsäure zu 6-Hydroxynikotinoylchlorid mit einem Säurechlorid ist an sich gemäss der CH-PS 664 754 bekannt.

Als Säurechloride sind für die erste Stufe beispielsweise Thionylchlorid, Phosphoroxychlorid oder Phosphorpentachlorid, vorzugsweise Thionylchlorid geeignet.

Die Säurechloride werden üblicherweise im Überschuss, bezogen auf das stöchiometrische Verhältnis, eingesetzt. Zweckmässig werden die Säurechloride in einer Menge von 5 bis 1 mol, vorzugsweise von 3,5 bis 1 mol, pro mol 6-Hydroxynikotinsäure, eingesetzt.

Zweckmässig wird die erste Stufe in Gegenwart eines tert. Amins als Katalysator durchgeführt. Als tert. Amine können beispielsweise Pyridine, die gegebenenfalls alkylsubstituiert sind, eingesetzt werden. Vorzugsweise wird Pyridin eingesetzt.

Die Katalysatoren werden zweckmässig in einer Menge von 0,01 bis 0,3 mol, bezogen auf 1 mol 6-Hydroxynikotinsäure, eingesetzt.

Die Umsetzungstemperatur liegt in der ersten Stufe zweckmässig in einem Bereich zwischen 0 und 80°C.

Als Lösungsmittel sind für die erste Stufe inerte Lösungsmittel, wie beispielsweise Methylenchlorid, Chloroform oder Tetrachlorkohlenstoff geeignet.

Es wurde nun jedoch gefunden, wenn man in der ersten Stufe als Lösungsmittel Acetonitril verwendet, dass man die Menge des für die Umsetzung nötigen Katalysators erheblich reduzieren kann. Demnach wird in einer vorzugsweisen Ausführungsform die erste Stufe in Acetonitril als Lösungsmittel durchgeführt.

Vorzugsweise wird dann der Katalysator in einer Menge von 0,001 bis 0,01 mol, bezogen auf 1 mol 6-Hydroxynikotinsäure, eingesetzt.

Zweckmässig wird dann die erste Stufe bei einer Temperatur von 0 bis 100°C, vorzugsweise von 60 bis 80°C durchgeführt werden.

Nach einer üblichen Umsetzungsdauer von 0,2 bis 2 h kann dann 6-Hydroxynikotinoylchlorid nach fachmännisch üblicher Methode isoliert werden.

Die zweite Stufe, die Umsetzung von 6-Hydroxy-nikotinoylchlorid zu 6-Hydroxynikotinsäurealdehyd erfolgt durch katalytische Hydrierung mit Wasserstoff.

Als Hydrierkatalysatoren können für die zweite Stufe Edelmetall-Katalysatoren, gegebenenfalls auf einem geeigneten Träger, angewendet werden.
Vorzugsweise wird als Hydrierkatalysator Palladium auf Aktivkohle angewendet, insbesondere 5 - 10 Gew.% Palladium auf Aktivkohle.
Die Hydrierkatalysatoren können in der zweiten Stufe in einer Menge von 0,01 bis 0,1 mol, vorzugsweise von 0,03 bis 0,05 mol, pro mol 6-Hydroxynikotinoylchlorid, angewendet werden.

Die zweite Stufe kann bei Normaldruck oder bei einem erhöhten H₂-Druck erfolgen. Vorzugsweise erfolg die zweite Stufe bei einem erhöhten H₂-Druck von 3 bis 20 bar.

Die Umsetzung in der zweiten Stufe kann mit oder ohne Zusatz einer sogenannten Hilfsbase erfolgen. Als Hilfsbasen können tert. Amine, wie beispielsweise Triethylamin oder 2,6-Lutidin angewendet werden. Vorzugsweise wird die zweite Stufe ohne Hilfsbase durchgeführt.

Zweckmässig erfolgt die zweite Stufe in einem inerten Lösungsmittel. Geeignete Lösungsmittel für die zweite Stufe sind beispielsweise Acetonitril, Aceton, Tetrahydrofuran und Essigester. Vorzugsweise wird in der zweiten Stufe als Lösungsmittel Acetonitril angewendet.

Die Umsetzung in der zweiten Stufe erfolgt zweckmässig bei einer Temperatur von 0 bis 150°C, vorzugsweise von 80 bis 130°C.

Nach einer üblichen Umsetzungsdauer von 2 bis 10 h kann dann nach der zweiten Stufe 6-Hydroxynikotinsäurealdehyd nach fachmännisch üblichen Methoden isoliert werden.

Die dritten Stufe, die Hydrierung des 6-Hydroxynikotinsäurealdehyds zum 2-Hydroxy-5-hydroxymethylpyridin erfolgt ebenfalls katalytisch mit Wasserstoff.

Als Hydrierkatalysatoren können für die dritte Stufe Edelmetall-, Edelmetalloxid-oder Raney-Katalysatoren, gegebenenfalls auf einem geeigneten Träger, angewendet werden. Vorzugsweise wird die dritte wie die zweite Stufe mit Palladium auf Aktivkohle, insbesondere mit 5 - 10 Gew.% Palladium auf Aktivkohle als Hydrierkatalysator angewendet. Die Menge des Hydrierkatalysators liegt zweckmässig in der dritten Stufe zwischen 0,001 und 0,01 mol, vorzugsweise zwischen 0,001 und 0,005 mol, pro mol 6-Hydroxynikotinsäurealdehyd.

Die dritte Stufe kann ebenfalls wie die zweite Stufe bei Normaldruck oder bei einem erhöhten H₂-Druck erfolgen. Vorzugsweise erfolgt die Hydrierung bei einem erhöhten H₂-Druck zwischen 2 und 20 bar.

Die dritte Stufe erfolgt zweckmässig in einem polaren Lösungsmittel. Als polare Lösungsmittel können beispielsweise Wasser, Methanol, Ethanol, 2-Propanol angewendet werden, vorzugsweise wird Wasser angewendet.

Die Umsetzungstemperatur liegt in der dritten Stufe zweckmässig zwischen 0 und 150°C, vorzugsweise zwischen 20 und 100°C.

Nach einer üblichen Umsetzungsdauer von 0,2 bis 2 h wird dann 2-Hydroxy-5-hydroxymethylpyridin nach fachmännisch üblichen Methoden isoliert.

Die vierte Stufe, die Chlorierung von 2-Hydroxy-5-hydroxymethylpyridin zu CCPM erfolgt mit fachmännisch üblichen Chlorierungsmitteln, wie beispielsweise Phosphorpentachlorid, Phosphoroxychlorid und Phosgen.

Üblicherweise wird das Chlorierungsmittel im Überschuss, bezogen auf 2-Hydroxy-5-hydroxymethylpyridin, eingesetzt, vorzugsweise in einer Menge von 2 bis 5 mol, pro mol 2-Hydroxy-5-hydroxymethylpyridin.

Als Lösungsmittel werden in der vierten Stufe zweckmässig inerte Lösungsmittel eingesetzt.

Als inerte Lösungsmittel kommen beispielsweise Chloroform, Methylenchlorid, Tetrachlorkohlenstoff oder Phosphoroxychlorid in Frage. Vorzugsweise wird die vierte Stufe gelöst in Phosphoroxychlorid durchgeführt.

Die Umsetzungstemperatur liegt in der vierten Stufe zweckmässig zwischen 0 und 150°C, vorzugsweise zwischen 80 und 120°C.

Nach einer üblichen Umsetzungsdauer von 2 bis 8 h wird dann CCPM nach fachmännisch üblichen Methoden in guter Ausbeute und Reinheit erhalten.

### Beispiel 1

### Herstellung von 6-Hydroxynikotinoylchlorid

13,92 g (0,1 mol) 6-Hydroxynikotinsäure und 0,04 g (0,5 mmol) Pyridin wurden in 60 ml Acetonitril auf 80°C erhitzt. Es wurden 12,49 g (0,105 mol) Thionylchlorid zugetropft, und die Reaktion wurde weitere 30 min bei 80°C gerührt. Nach dem Abkühlen wurde der Niederschlag abfiltriert, zweimal mit je 10 ml kaltem Acetonitril gewaschen und im Vakuum getrocknet. Man erhielt 13,38 g (0,085 mol) 6-Hydroxynikotinoylchlorid als leicht gelbliches Pulver, entsprechend einer Ausbeute von 85%, bezogen auf 6-Hydroxynikotinsäure.
¹H-NRM: (CDCl₃, 300 MHz) δ in ppm 8,44 (d, J = 2,6 Hz, 1H, H-2); 8,01 (dd, J = 2,6 Hz, J = 9,7 Hz, 1H, H-4); 6,62 (d, J = 9,7 Hz, 1H, H-5)

### Beispiel 2

### Herstellung von 6-Hydroxynikotinsäurealdehyd

13,35 g (0,086 mol) 6-Hydroxynikotinoylchlorid in 650 ml Acetonitril wurden in einem Autoklav mit 2,7 g 5% Pd/C-Katalysator bei 80°C und 10 bar H₂ hydriert. Nach Ende der Reaktion(4 h)wurde der Katalysator abfiltriert und mehrmals mit heissem Wasser gewaschen. Die vereinten Filtrate wurden eingeengt, und der Rückstand aus Wasser umkristallisiert. Es wurden 7,94 g (64 mmol) 6-Hydroxynikotinsäurealdehyd als farblose Kristalle erhalten, entsprechend einer Ausbeute von 74%, bezogen auf 6-Hydroxynikotinoylchlorid.
Smp.: 219°C
¹H-NRM: (CDCl₃, 300 MHz) δ in ppm: 12,31 (s, br, 1H, OH) 9,59 (s, 1H, CHO); 8,25 (D, j = 2,3 Hz, 1H, H-2); 7,75 (dd, J = 9,6 Hz, J = 2,4 Hz, 1H, H-4); 6,41 (d, J = 9,6 Hz, 1H, H-5).

### Beispiel 3

### Herstellung von 2-Hydroxy-5-hydroxymethyl-pyridin

1,0 g (8,1 mmol) 6-Hydroxynikotinsäurealdehyd in 80 ml Wasser wurden in einem Autoklav mit 50 mg 5% Pd/C-Katalysator bei Raumtemperatur und 10 bar H₂ hydriert. Nach dem Ende der Reaktion(1 h)wurde der Katalysator abfiltriert und das Filtrat eingeengt. Man erhielt 0,96 g eines farblosen Feststoffs, entsprechend einer Ausbeute von 94%, bezogen auf 6-Hydroxynikotinsäurealdehyd.
¹H-NMR (D₂O, 300 MHz) δ in ppm 7,73 (dd, J = 2,2 Hz, J = 9,3, 1H, H-4); 7,52 (d, J = 2,2 Hz, 1H, H-6); 6,63 (d, J = 9,3 Hz, 1H, H-3); 4,48 (s, CH₂OH, 1H).

### Beispiel 4

### Herstellung von CCPM

Eine Lösung von 2,5 g (20 mmol) 2-Hydroxy-5-hydroxymethylpyridin und 4,16 g Phosphorpentachlorid in 10 ml Phosphorylchlorid wurde 6 h bei 105°C gerührt. Nach dem Abkühlen wurden 50 ml Chloroform zugegeben, das überschüssige Chlorierungsreagens durch vorsichtige Zugabe von Wasser hydrolisiert. Die organische Phase wurde mit NaHCO₃-Lösung gewaschen, über NaSO₄ getrocknet und eingeengt. Die Destillation des öligen Rückstands bei 16 mm und 120°C ergab 3,08 g CCPM, als farbloses Öl, das beim Abkühlen fest wurde. Dies entspricht einer Ausbeute von 95%, bezogen auf 2-Hydroxy-5-hydroxymethylpyridin.
¹H-NMR: (CDCl₃, 300 MHz) δ in ppm 8,28 (d, J = 2,3 Hz, 1H, H-6); 7,72 (dd, J = 8,2 Hz, J = 2,3 Hz, H-4); 7,34 (d, J = 8,2 Hz, 1H, H-3); 4,58 (s, CH₂Cl, 2H).

## Patentansprüche

1. Verfahren zur Herstellung von 2-Chlor-5-chlormethylpyridin der Formel 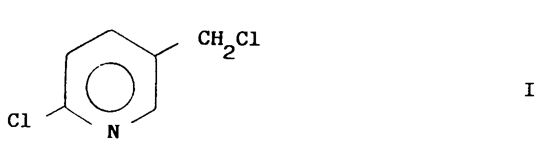 dadurch gekennzeichnet, dass man in der ersten Stufe 6-Hydroxynikotinsäure der Formel 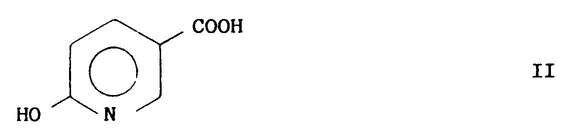 mit einem Säurechlorid zu 6-Hydroxynikotinoylchlorid der Formel 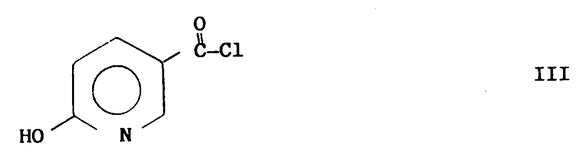 umsetzt, dieses in der zweiten Stufe katalytisch mit Wasserstoff zu 6-Hydroxynikotinsäurealdehyd der Formel 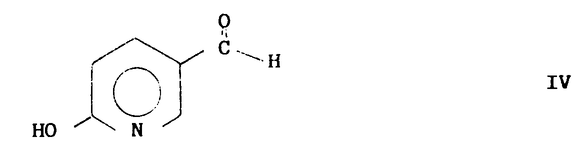 hydriert, dann in der dritten Stufe katalytisch mit Wasserstoff zu 2-Hydroxy-5-hydroxymethylpyridin der Formel 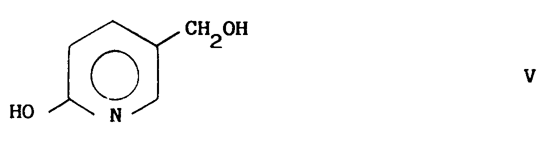 hydriert und dieses dann in der vierten Stufe zum Endprodukt gemäss Formel I chloriert.

2. Verfahren nach Patentanspruch 1, dadurch gekennzeichnet, dass man in der ersten Stufe als Säurechlorid Thionylchlorid und als Lösungsmittel Acetonitril verwendet.

3. Verfahren nach Patentanspruch 2, dadurch gekennzeichnet, dass man die Umsetzung in der ersten Stufe in Gegenwart eines tert. Amins, als Katalysator, in einer Menge von 0,001 bis 0,01 mol, pro mol 6-Hydroxynikotinsäure durchführt.

4. Verfahren nach mindestens einem der Patentansprüche 2 und 3, dadurch gekennzeichnet, dass man die Umsetzung in der ersten Stufe bei einer Temperatur von 0 bis 100°C durchführt.

5. Verfahren nach mindestens einem der Patentansprüche 1 bis 4, dadurch gekennzeichnet, dass man die Hydrierung in der zweiten Stufe bei einem Druck von 3 bis 20 bar und bei einer Temperatur von 0 bis 150°C durchführt.

6. Verfahren nach mindestens einem der Patentansprüche 1 bis 5, dadurch gekennzeichnet, dass man als Hydrierkatalysator in der zweiten und dritten Stufe Palladium auf Kohle anwendet.

7. Verfahren nach mindestens einem der Patentansprüche 1 bis 6, dadurch gekennzeichnet, dass man die Umsetzung in der dritten Stufe bei einem Druck von 2 bis 20 bar und bei einer Temperatur von 0 bis 150°C durchführt.

8. Verfahren nach mindestens einem der Patentansprüche 1 bis 7, dadurch gekennzeichnet, dass man die Chlorierung in der vierten Stufe mit Phosphorpentachlorid, Phosphoroxychlorid oder mit Phosgen durchführt.

9. Verfahren nach mindestens einem der Patentansprüche 1 bis 8, dadurch gekennzeichnet, dass man die Chlorierung in der vierten Stufe bei einer Temperatur von 0 bis 150°C durchführt.

## Claims

1. A process for the preparation of 2-chloro-5-chloromethyl pyridine of the formula 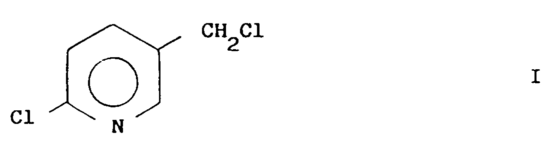 characterized in that, in a first step, 6-hydroxynicotinic acid of the formula 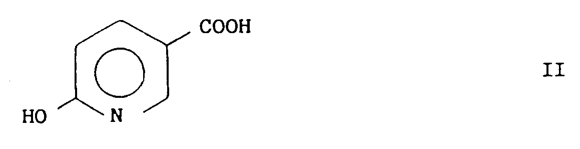 is reacted with an acid chloride to give 6-hydroxynicotinic acid chloride of the formula 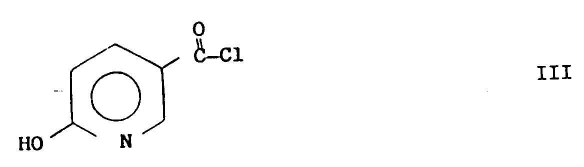 which, in a second step, is catalytically hydrogenated with hydrogen to give 6-hydroxynicotinic aldehyde of the formula 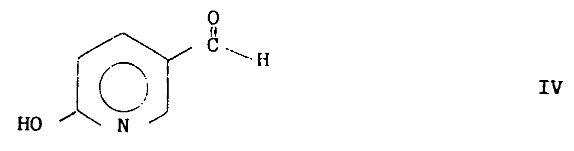 which, in a third step, is subsequently catalytically hydrogenated with hydrogen to give 2-hydroxy-5-hydroxymethyl pyridine of the formula 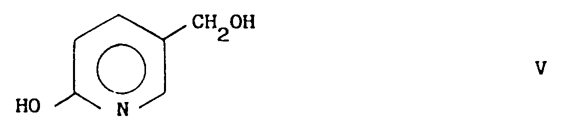 which, in a fourth step, is subsequently chlorinated to give the end product of the formula I.

2. The process of patent claim 1, characterized in that thionyl chloride is used as the acid chloride of the first step and acetonitrile is used as a solvent.

3. The process of patent claim 2, characterized in that the reaction of the first step is carried out in the presence of a tertiary amine, as a catalyst, in an amount of from 0.001 to 0.01 moles per mole of 6-hydroxynicotinic acid.

4. The process of at least one of the patent claims 2 and 3, characterized in that the reaction of the first step is carried out at a temperature of from 0 to 100°C.

5. The process of at least one of the patent claims 1 to 4, characterized in that the hydrogenation of the second step is carried out at a pressure of from 3 to 20 bar and at a temperature of from 0 to 150°C.

6. The process of at least one of the patent claims 1 to 5, characterized in that palladium on a carbon support is used as the hydrogenation catalyst of the second and third step.

7. The process of at least one of the patent claims 1 to 6, characterized in that the reaction of the third step is carried out at a pressure of from 2 to 20 bar and at a temperature of from 0 to 150°C.

8. The process of at least one of the patent claims 1 to 7, characterized in that the chlorination of the fourth step is effected with phosphorus pentachloride, phosphorus oxychloride or phosgene.

9. The process of at least one of the patent claims 1 to 8, characterized in that the chlorination of the fourth step is carried out at a temperature of from 0 to 150°C.

## Revendications

1. Procédé pour la préparation de 2-chloro-5-chlorométhylpyridine de la formule 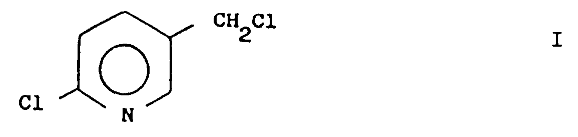 caractérisé en ce que l'on transforme dans la première étape l'acide 6-hydroxynicotinique de la formule 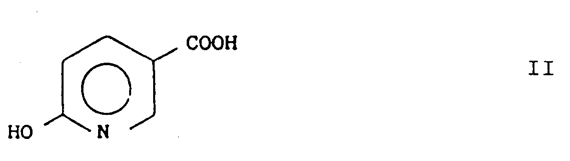 avec un chlorure d'acide en 6-hydroxynicotinoyl-chlorure de la formule 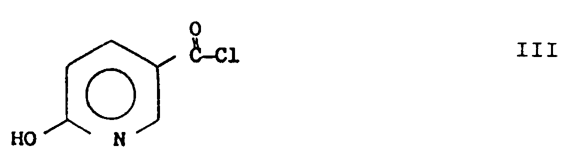 que l'on hydrogène celui-ci dans la deuxième étape catalytiquement avec de l'hydrogène pour donner de l'acide 6-hydroxynicotinique de la formule 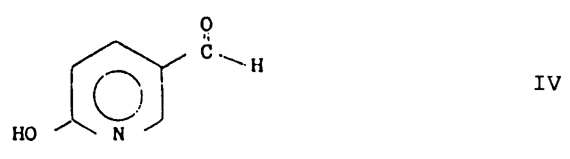 puis, dans la troisième étape que l'on hydrogène catalytiquement avec de l'hydrogène pour donner de la 2-hydroxy-5-hydroxyméthylpyridine de la formule 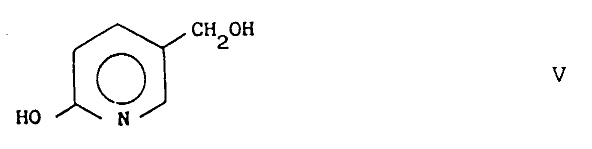 et que l'on chlorure celle-ci dans la quatrième étape en produit final selon la formule 1.

2. Procédé selon la revendication 1 caractérisé en ce que dans la première étape on utilise comme chlorure d'acide le chlorure de thionyle et comme solvant l'acétonitrile.

3. Procédé selon la revendication 2 caractérisé en ce que dans la première étape on conduit la réaction en présence d'un tert.amine en tant que catalyseur, dans une quantité de 0,001 jusqu'à 0,01 mole, par mole, de l'acide 6-hydroxynicotinique.

4. Procédé selon au moins l'une des revendications 2 et 3 caractérisé en ce que dans la première étape on conduit la réaction à une température de 0 à 100 °C.

5. Procédé selon au moins l'une des revendications 1 à 4, caractérisé en ce que dans la deuxième étape on conduit l'hydrogénation à une pression de 3 à 20 bars et à une température de 0 à 150 °C.

6. Procédé selon au moins l'une des revendications 1 à 5, caractérisé en ce qu'en tant que catalyseur d'hydrogénation dans la deuxième étape et dans la troisième étape on utilise du palladium sur du charbon actif.

7. Procédé selon au moins l'une des revendications 1 à 6, caractérisé en ce que dans la troisième étape on conduit la réaction à une pression de 2 à 20 bars et à une température de 0 à 150°C.

8. Procédé selon au moins l'une des revendications 1 à 7, caractérisé en ce que l'on conduit la chloruration dans la quatrième étape, avec du pentachlorure de phosphore, de l'oxychlorure de phosphore ou avec du phosgène.

9. Procédé selon au moins l'une des revendications 1 à 8, caractérisé en ce que dans la quatrième étape la chloruration s'effectue à une température de 0 à 150 °C.
